# EUROPÄISCHE PATENTANMELDUNG

(11) **EP 0 735 021 A2**
(43) Veröffentlichungstag der Anmeldung: **02.10.1996**
(21) Anmeldenummer: 96104372.6
(22) Anmeldetag: 20.03.1996
(51) Int. Cl.: C07C 303/32, C07C 309/12

(54) **Verfahren zur Herstellung von Acyloxyalkansulfonaten**

(30) Priorität: 29.03.1995 DE 19511460
(71) Anmelder: HOECHST AKTIENGESELLSCHAFT, 65929 Frankfurt am Main (DE)
(72) Erfinder: Bühring, Dirk, Dr., Rua Particular casa 35, 08613-010 Suzano - SP (BR)

(57) **Zusammenfassung**

Die Acyloxyalkansulfonate werden erfindungsgemäß durch Veresterung von verzweigten Fettsäuren mit Hydroxyalkansulfonaten hergestellt. Aufgrund der speziellen Fettsäure ist der Einsatz eines Konsistenzreglers nicht erforderlich. Es werden hochkonzentrierte Acyloxyalkansulfonate erhalten, die als weitere vorteilhafte Eigenschaft gute Wasserlöslichkeit besitzen.

## Beschreibung

Die Erfindung betrifft ein Verfahren zur Herstellung von Acyloxyalkansulfonaten mit verbesserten Eigenschaften durch Veresterung von Fettsäuren mit Hydroxyalkansulfonaten.

Acyloxyalkansulfonate sind wertvolle anionische Tenside, die vor allem zur Bereitung von Syndet-Seifen, kosmetischen Mitteln und Reinigungsformulierungen eingesetzt werden. Ihre Herstellung erfolgt vorteilhaft durch Veresterung von mindestens einer Fettsäure mit mindestens einem Hydroxyalkansulfonat (Direktveresterung). Ein solches Verfahren ist zum Beispiel in EP-A-0 585 071 (US-A-5 384 421) beschrieben. Dabei werden die Fettsäure und das Salz der Hydroxyalkansulfonsäure in Gegenwart eines Veresterungskatalysators und eines Konsistenzreglers bei einer Temperatur von 180 bis 240 °C umgesetzt unter gleichzeitiger Entfernung von vorhandenem Wasser. Als Konsistenzregler werden bestimmte Paraffine eingesetzt. Der Einsatz derartiger Verbindungen ist erforderlich, weil mit fortschreitender Veresterung das Reaktionsgemisch hochviskos wird. Mit Konsistenzreglern wird zwar eine niedrigere Viskosität des Reaktionsgemisches erreicht und die Umsetzung erleichtert, das Veresterungsprodukt enthält aber die eingesetzten Verbindungen, wodurch das angestrebte Acyloxyalkansulfonat mehr oder weniger verdünnt erhalten wird.

Es wurde nun überraschenderweise gefunden, daß man die in Rede stehende Direktveresterung ohne Konsistenzregler durchführen kann und damit ein Veresterungsprodukt mit einem hohen Gehalt an Acyloxyalkansulfonat erhält, wenn man als Fettsäuren solche mit einem verzweigten (ungeraden) Kohlenwasserstoffrest einsetzt. Es ist ein unerwartetes Ergebnis, daß die Umsetzung von Hydroxyalkansulfonaten gerade mit verzweigten Fettsäuren ohne Konsistenzregler durchführbar ist. Auf diese Weise ist es möglich, hochkonzentrierte Acyloxyalkansulfonate herzustellen, die als weitere unerwartete Eigenschaften gute Wasserlöslichkeit, hohes Schaumvermögen und gute Hartwasserstabilität aufweisen.

Das erfindungsgemäße Verfahren zur Herstellung von Acyloxyalkansulfonaten mit verbesserten Eigenschaften durch Veresterung von Fettsäuren mit Hydroxyalkansulfonaten ist dadurch gekennzeichnet, daß man mindestens eine Fettsäure der Formel 1 RCOOH (1), worin R ein verzweigter Kohlenwasserstoffrest mit 5 bis 31 Kohlenstoffatomen ist oder eine Mischung aus verzweigten und unverzweigten Kohlenwasserstoffresten mit jeweils 5 bis 31 Kohlenstoffatomen ist, wobei die Menge an unverzweigten Resten höchstens 50 Gew.-% beträgt, mit mindestens einem Hydroxyalkansulfonat der Formel 2 HO-R¹-SO₃X (2), worin R¹ ein C₂ bis C₄-Alkylen oder ein divalenter Di-C₂ bis C₄-alkyletherrest ist und X ein Alkalimetall oder Ammonium ist, in Gegenwart eines Veresterungskatalysators und in Abwesenheit eines Konsistenzreglers bei einer Temperatur von 180 bis 250 °C unter Entfernen des anwesenden Wassers verestert, wobei ein Produkt mit einem hohen Gehalt an Acyloxyalkansulfonat erhalten wird.

Beim erfindungsgemäßen Verfahren werden also ausgewählte Fettsäuren eingesetzt, das heißt solche mit einem gesättigten oder ungesättigten, verzweigten Kohlenwasserstoffrest mit (insgesamt) 5 bis 31 Kohlenstoffatomen. Der Kohlenwasserstoffrest kann eine oder mehrere Verzweigungen aufweisen, im allgemeinen enthält er nur eine Verzweigung, und zwar in α-Stellung zur Carboxylgruppe. Die Verzweigung oder die Verzweigungen selbst können kurz oder lang, unverzweigt oder verzweigt, vorzugsweise unverzweigt, gesättigt oder ungesättigt, vorzugsweise gesättigt, sein. Der verzweigte Kohlenwasserstoffrest R in Formel 1 ist bevorzugt ein Rest der nachstehenden Formel 1a 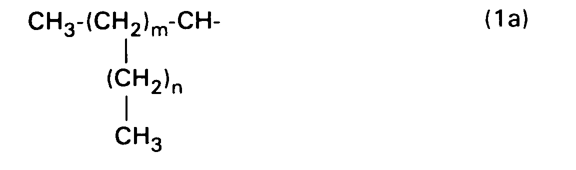 worin m eine ganze Zahl von 4 bis 18 ist, vorzugsweise 4 bis 14, und n 0 oder eine ganze Zahl von 1 bis 10 ist, vorzugsweise 0 oder eine ganze Zahl von 1 bis 6. Die erfindungsgemäß bevorzugten Fettsäuren entsprechen also der nachstehenden Formel 1b 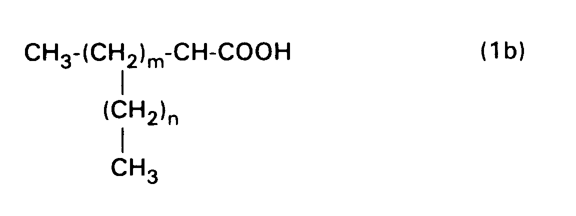 worin m und n die angegebenen Bedeutungen haben. Die erfindungsgemäß einzusetzenden verzweigten (ungeraden) Fettsäuren sind bekannt und im Handel erhältlich. Verzweigte Carbonsäuren können zum Beispiel aus inner-Olefinen hergestellt werden, indem diese durch Oxo-Synthese zum Aldehyd umgesetzt und die Aldehydverbindungen zur Carbonsäure oxidiert werden. Beispiele für geeignete verzweigte Fettsäuren sind: 2-Ethylhexansäure, 2-Pentyloctansäure, 2-Butylnonansäure, 2-Propyldecansäure, 2-Ethylundecansäure, 2-Butylundecansäure, 2-Methyldodecansäure, 2-Ethyltridecansäure und 2-Methyltetradecansäure und Mischungen davon.

Erfindungsgemäß können auch Mischungen aus mindestens einer verzweigten Fettsäure und mindestens einer unverzweigten (geraden) Fettsäure eingesetzt werden, wobei der Anteil an unverzweigten Fettsäuren höchstens 50 Gew.-% beträgt, Gewichtsprozente bezogen auf die Mischung. Ebenso wie die verzweigten Fettsäuren können auch die unverzweigten gesättigt oder ungesättigt sein. Im Falle von unverzweigten Fettsäuren ist der Rest R vorzugsweise C₅ bis C₂₁-Alkyl oder C₅ bis C₂₁-Alkenyl oder eine Mischung davon. Die Alkenylreste sind vorzugsweise ein- bis dreifach ungesättigt. Als Beispiele für unverzweigte Fettsäuren seien genannt Capronsäure, Caprinsäure, Laurinsäure, Myristinsäure, Stearinsäure, Arachinsäure, Ölsäure, Linolsäure, Linolensäure, Cocosfettsäure und Talgfettsäure.

Das Hydroxyalkansulfonat, das mit der verzweigten Fettsäure oder mit der genannten Mischung aus verzweigten und unverzweigten Fettsäuren umgesetzt wird, ist nicht kritisch. Bevorzugte Salze von Hydroxyalkansulfonsäuren sind solche der Formel 2, worin R¹ -CH₂CH₂-, -(CH₂)₃-, -CH₂CH(CH₃)- oder -CH₂CH₂OCH₂CH₂- ist, wobei Ethylen besonders bevorzugt ist, und das Gegenion X NH₄ ist oder ein Alkalimetall, vorzugsweise Natrium oder Kalium. Das erfindungsgemäß besonders bevorzugte Hydroxyalkansulfonat ist Kalium- oder Natriumisethionat. Die Hydroxyalkansulfonsäuresalze können als solche eingesetzt werden, bevorzugt werden sie in Form einer wäßrigen Lösung, im allgemeinen als 40 bis 65gew.%ige Lösung, eingesetzt.

Die erfindungsgemäße Umsetzung von Fettsäure und Hydroxyalkansulfonat wird in Gegenwart eines Katalysators durchgeführt. Geeignete Veresterungskatalysatoren sind in der genannten EP-A-0 585 071 ausführlich beschrieben, die hier miteinbezogen wird. Es handelt sich um Alkansulfonsäuren, Hydroxyalkansulfonsäuren, Arylsulfonsäuren, anorganischen Säuren wie Schwefelsäure, Phosphorsäure, phosphorige Säure, Borsäure oder deren Anhydride, Schwermetallsalze wie Zinksulfat, Zirkoniumsulfat, Zinkisethionat, Zinkborat, Aluminiumsulfat, Titansulfat oder Wolframphosphat, Metalloxide wie Zinkoxid, Aluminiumoxid, Magnesiumoxid, Ceroxid, Zirkoniumoxid oder Lanthanoxid, ferner um Mischungen von zwei oder mehreren der genannten Katalysatoren und um Seifen, die aus Schwermetallen und Metalloxiden gebildet werden. Ein besonders bevorzugter Veresterungskatalysator ist Zinkoxid. Der Veresterungskatalysator wird in einer Menge von im allgemeinen 0,05 bis 2 Gew.-%, vorzugsweise 0,05 bis 1 Gew.-%, eingesetzt, Gewichtsprozente bezogen auf Fettsäure und Hydroxyalkansulfonsäuresalz.

Die Umsetzung von Fettsäure und Hydroxyalkansulfonsäuresalz, im allgemeinen im Molverhältnis von 1 : 1 bis 2 : 1, vorzugsweise etwa 1 : 1, wird erfindungsgemäß bei einer Temperatur von 180 bis 250 °C durchgeführt. Der bevorzugte Temperaturbereich liegt bei 200 bis 240 °C. Das gegebenenfalls mit den Ausgangskomponenten in die Reaktionsmischung eingebrachte Wasser und das durch die Veresterungsreaktion entstehende Wasser wird kontinuierlich aus der Reaktionsmischung ausgetragen. Die Reaktionsmischung ist aufgrund der eingesetzten speziellen Fettsäure bis zum Ende der Umsetzung, auch bei 100%igem Umsatz, homogen, relativ niedrigviskos und gut rührbar; ein Konsistenzregler ist nicht erforderlich. Die Zeit bis zum angestrebten Umsatz von Fettsäure oder von Hydroxyalkansulfonat liegt bei etwa 4 bis 8 Stunden. Im allgemeinen wird man, zum Beispiel aus Zeitgründen, 100%igen Umsatz nicht anstreben, sondern bei einem niedrigeren Prozentsatz, zum Beispiel bei 75 bis 90 Gew.-% Acyloxyalkansulfonat, die Veresterungsreaktion abbrechen.

Das erfindungsgemäße Verfahren kann im einzelnen zum Beispiel in der Weise durchgeführt werden, daß man - bei Atmosphärendruck - die Fettsäure, das Salz der Hydroxyalkansulfonsäure und den Veresterungskatalysator in einem Reaktionsgefäß vorlegt und die Mischung unter Rühren auf die angegebene Temperatur erhitzt. Vorhandenes Wasser destilliert bereits während des Erhitzens der Reaktionsmischung und dann weiter während der laufenden Veresterungsreaktion kontinuierlich ab. Das erfindungsgemäße Verfahren kann auch nach der in EP-A-0 585 071 beschriebenen Methode durchgeführt werden. Hier wird die Veresterungsreaktion teils bei Atmosphärendruck und teils unter Anwendung eines Vakuums zur schnelleren Austragung des Wassers durchgeführt. Nach Erreichen des angestrebten Umsetzungsgrades wird die Veresterungsreaktion zum Beispiel durch Abkühlen beendet. Das erhaltene Reaktionsprodukt ist bei Raumtemperatur flüssig oder fest. Eine Konfektionierung des bei Raumtemperatur festen Produktes kann man zum Beispiel mit Hilfe einer Schuppenwalze oder eines Kühlbandes durchführen.

Mit dem erfindungsgemäßen Verfahren, das auch großtechnisch durchführbar ist, können konzentrierte Acyloxyalkansulfonate hergestellt werden. Sie weisen als weitere vorteilhafte Eigenschaften gute Wasserlöslichkeit, starke Schaumbildung und gute Hartwasserstabilität sowie ein gut wirkendes Hautgefühl auf. Die erfindungsgemäß erhaltenen Produkte eignen sich deshalb vor allem auch für wäßrige Formulierungen. Durch die Direktveresterung (Direktkondensation) kann auf die Verwendung von Fettsäurechloriden verzichtet werden, die sonst in einem gesonderten Schritt aus Fettsäure hergestellt werden müßten.
Die Verwendung von Kosistenzreglern und/oder Verdünnungsmitteln, die in der Regel keine Wertstoffe für zum Beispiel Reinigungsformulierungen und kosmetischen Mitteln darstellen, ist nicht notwendig. Beim erfindungsgemäßen Verfahren besteht also die Reaktionsmischung im wesentlichen lediglich aus verzweigter Fettsäure, Hydroxyalkansulfonat und Veresterungskatalysator. Das feste oder flüssige Reaktionsprodukt enthält, wie oben erwähnt, im allgemeinen 75 bis 90 Gew.-% Acyloxyalkansulfonat, bezogen auf das feste oder flüssige Gesamtprodukt. Die erfindungsgemäß erhaltenen Salze von Acyloxyalkansulfonsäuren entsprechen der nachstehenden Formel 3 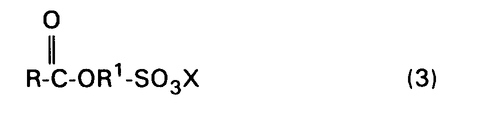 worin R, R¹ und X die angegebenen Bedeutungen haben.

Die Erfindung wird nun an Beispielen noch näher erläutert. Prozentangaben beziehen sich auf das Gewicht, wenn keine anderen Angaben gemacht sind.

### Beispiel 1

In einem 2-l-Planschliffbecher mit Ankerrührer, absteigender Destillationsbrücke, Innenthermometer und Stickstoffeinleitung werden 639 g (3 mol) Isotridecansäure mit einem mittleren Molekulargewicht von 214 (Zusammensetzung im einzelnen siehe unten), 775 g von einer wäßrigen 57%igen Natriumisethionat-Lösung (das sind 3 mol Natriumisethionat) und 3,0 g Zinkoxid vorgelegt. Die Mischung wird auf 235 °C erwärmt und bei dieser Temperatur gehalten. Das in die Mischung eingebrachte und das bei der Direktkondensation gebildete Wasser destilliert
kontinuierlich ab. Bei einem Natriumisotridecanoylisethionat-Gehalt von 79 % wird die Reaktion abgebrochen. Das Reaktionsprodukt wird auf 120 °C abgekühlt und zum Erkalten auf ein Blech gegossen. Es besteht im wesentlichen aus 79 % Natriumisotridecanoylisethionat (Epton-Titration),
10 % Laurinsäure (potentiometrische Titration) und 11 % Natriumisethionat (aus dem Umsatz berechnet).

Zusammensetzung der Isotridecansäure (Hauptbestandteile): 19 % 2-Pentyloctansäure, 18 % 2-Butylnonansäure, 17 % 2-Propyldecansäure,
17 % 2-Ethylundecansäure, 25 % 2-Methyldodecansäure, 3 % n-Tridecansäure.

### Beispiel 2

In der Einrichtung vom Beispiel 1 werden 554 g (2,6 mol) Isotridecansäure (Zusammensetzung wie im Beispiel 1), 517 g von einer wäßrigen 57%igen Natriumisethionat-Lösung (2 mol Natriumisethionat) und 2,0 g Zinkoxid vorgelegt. Der Ansatz wird auf 235 °C erwärmt und bei dieser Temperatur gehalten, wobei anwesendes Wasser kontinuierlich abdestilliert. Bei einem Natriumisotridecanoylisethionat-Gehalt von 77 % wird Vakuum (0,5 mbar) angelegt, und bei einer Temperatur von weiterhin 235 °C werden 97 g der im Überschuß eingesetzten Isotridecansäure abdestilliert. Das Produkt wird auf 150 °C abgekühlt und zum Erkalten auf ein Blech gegossen. Das Endprodukt besteht zu 89 % aus Natriumisotridecanoylisethionat (Epton-Titration), die Säurezahl liegt bei 19 mg KOH/g (potentiometrische Titration).

### Beispiel 3

In der Einrichtung vom Beispiel 1 werden 391 g (1,8 mol) Isotridecansäure (Zusammensetzung wie im Beispiel 1), 353 g (1,8 mol) Natrium-2-(2-hydroxyethoxy)ethansulfonat und 2,0 g Zinkoxid vorgelegt. Der Ansatz wird auf 235 °C erwärmt und bei dieser Temperatur gehalten. Das bei der Direktkondensation gebildete Wasser destilliert kontinuierlich ab. Bei einem Natrium-2-(2-isotridecanoyloxyethoxy)ethansulfonat-Gehalt von 82 % wird die Reaktion abgebrochen und das Produkt zum Erkalten auf ein Blech gegossen. Das Reaktionsprodukt besteht im wesentlichen aus 82 % Natrium-2-(2-isotridecanoyloxyethoxy)ethansulfoant (Epton-Titration), 6 % Isotridecansäure (potentiometrische Titration) und 12 % Natrium-2-(2-hydroxyethoxy)ethansulfonat (aus dem Umsatz berechnet).

### Beispiel 4

In der Einrichtung vom Beispiel 1 werden 561 g (2,5 mol)
C₁₃ bis C₁₅-Carbonsäure mit einem mittleren Molekulargewicht von 228 (Zusammensetzung im einzelnen siehe unten), 511 g von einer wäßrigen 57%igen Natriumisethionat-Lösung (2 mol Natriumisethionat) und 1,6 g Zinkoxid vorgelegt. Die Mischung wird auf 220 °C erwärmt und bei dieser Temperatur gehalten. Das in die Mischung eingebrachte und das bei der Direktkondensation gebildete Wasser destilliert kontinuierlich ab. Bei einem Produktgehalt von 77 % wird Vakuum (0,5 mbar) angelegt, und bei einer Temperatur von weiterhin 220 °C werden 97 g der im Überschuß eingesetzten Carbonsäure abdestilliert. Das Reaktionsprodukt wird zum Erkalten auf ein Blech gegossen. Es besteht zu 89 % aus Natrium-C₁₃ bis C₁₅-acylisethionat (Epton-Titration), die Säurezahl liegt bei 10 mg KOH/g (potentiometrische Titration).

Zusammensetzung der C₁₃ bis C₁₅-Carbonsäure
(Hauptbestandteile mit einer Menge von > 1 %): 1,3 % 2-Propyldecansäure, 3,2 % 2-Ethylundecansäure, 19,3 % 2-Methyldodecansäure, 34,2 % n-Tridecansäure, 1,4 % 2-Butylundecansäure, 2,6 % 2-Ethyltridecansäure, 12,5 % 2-Methyltetradecansäure, 10,6 % n-Pentadecansäure.

### Test auf Wasserlöslichkeit und Schaumbildung:

1. Die erfindungsgemäßen Produkte der Beispiele 1 bis 4 und ein Produkt des Standes der Technik, und zwar Natriumcocoylisethionat mit 66 % waschaktiver Substanz und circa 28 % freier Fettsäure (Vergleich), werden bezüglich Wasserlöslichkeit getestet, indem geprüft wird, bis zu welcher maximalen Konzentration in entsalztem Wasser bei 20 °C eine klare Lösung erhalten wird. Die Werte sind in der nachstehenden Tabelle 1 zusammengefaßt:

**Tabelle 1**

| Wasserlöslichkeit (klare Lösungen, 20 °C, entsalztes Wasser) | | | | |
|---|---|---|---|---|
| Vergleich | Beispiel 1 | Beispiel 2 | Beispiel 3 | Beispiel 4 |
| < 1 % | 20 % | 30 % | 30 % | 10 % |

Es zeigt sich, daß mit dem Vergleichsprodukt eine klare Lösung nur dann erhalten wird, wenn die Menge an Produkt unter 1 % liegt, während bei den erfindungsgemäßen Produkten Konzentrationen von bis zu 30 % möglich sind, Prozente bezogen auf die Lösung.
2. Die erfindungsgemäßen Produkte der Beispiele 1 bis 3 und zwei Produkte des Standes der Technik, und zwar Natriumcocoylisethionat mit 66 % waschaktiver Substanz und circa 28 % freier Fettsäure (Vergleich 1) und Natriumcocoylisethionat mit > 90 % waschaktiver Substanz und < 5 % freier Fettsäure (Vergleich 2), werden bezüglich Ross/Miles-Schaum in Wasser mit 15 deutschen Härtegraden (15° dH), 37 °C und pH 7 bei einer Produktmenge von jeweils 1 % und 0,03 % (Prozente bezogen auf die wäßrige Lösung) getestet. Die Werte der Anfangsschaumhöhe (das ist die Schaumhöhe unmittelbar nach Ausladen der Lösung) und der Schaumhöhe nach 5 Minuten in mm sind in der nachstehenden Tabelle 2 zusammengefaßt:

**Tabelle 2**

| Ross/Miles-Schaum, pH 7, 15° dH, 37 °C: Anfangsschaumhöhe und Schaumhöhe nach 5 Minuten in mm | | | | | |
|---|---|---|---|---|---|
| Gehalt Aktivsubs tanz | Vergleic h 1 | Vergleic h 2 | Beispiel 1 | Beispiel 2 | Beispiel 3 |
| 1 % | | | | | |
| | 270/25 | 270/24 | 270/26 | 270/27 | 270/26 |
| | 0 | 0 | 0 | 0 | 0 |
| 0,03 % | 10/0 | 40/0 | | | |
| | | | 190/19 | 190/18 | 190/19 |
| | | | 0 | 0 | 0 |

3. Das erfindungsgemäße Produkt des Beispiels 1 und ein Produkt des Standes der Technik, und zwar Natriumcocoylisethionat mit 66 % waschaktiver Substanz und circa 28 % freier Fettsäure (Vergleich), werden bezüglich Schlagschaum in Wasser mit 15 deutschen Härtegraden (15° dH), 37 °C und pH 7 bei einer Produktmenge von jeweils 1 g/l getestet. Die Schaumwerte (Schaumvolumen) in ml am Anfang (das heißt unmittelbar nach Beendigung des Schlagvorganges) und nach 5 Minuten sind in der folgenden Tabelle 3 zusammengefaßt:

**Tabelle 3**

| Schlagschaum, pH 7, 15° dH, 1 g Substanz/l, 37 °C: Anfangsschaumvolumen und Schaumvolumen nach 5 Minuten in ml | |
|---|---|
| Vergleich | Beispiel 1 |
| 50/20 | 140/100 |

## Patentansprüche

1. Verfahren zur Herstellung von Acyloxyalkansulfonaten mit verbesserten Eigenschaften durch Veresterung von Fettsäuren mit Hydroxyalkansulfonaten, dadurch gekennzeichnet, daß man mindestens eine Fettsäure der Formel 1 RCOOH (1), worin R ein verzweigter Kohlenwasserstoffrest mit 5 bis 31 Kohlenstoffatomen ist oder eine Mischung aus verzweigten und unverzweigten Kohlenwasserstoffresten mit jeweils 5 bis 31 Kohlenstoffatomen ist, wobei die Menge an unverzweigten Resten höchstens 50 Gew.-% beträgt, mit mindestens einem Hydroxyalkansulfonat der Formel 2 HO-R¹-SO₃X (2), worin R¹ ein C₂ bis C₄-Alkylen oder ein divalenter Di-C₂ bis C₄-alkyletherrest ist und X ein Alkalimetall oder Ammonium ist, in Gegenwart eines Veresterungskatalysators und in Abwesenheit eines Konsistenzreglers bei einer Temperatur von 180 bis 250 °C unter Entfernen des anwesenden Wassers verestert, wobei ein Produkt mit einem hohen Gehalt an Acyloxyalkansulfonat erhalten wird.

2. Verfahren nach Anspruch 1, dadurch gekennzeichnet, daß R ein verzweigter Rest der nachstehenden Formel 1a ist 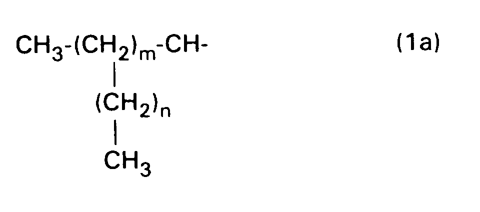 worin m eine ganze Zahl von 4 bis 18 und n 0 oder eine ganze Zahl von 1 bis 10 ist.

3. Verfahren nach Anspruch 2, dadurch gekennzeichnet, daß in Formel 1a m eine ganze Zahl von 4 bis 14 und n 0 oder eine ganze Zahl von 1 bis 6 ist.

4. Verfahren nach einem oder mehreren der Ansprüche 1 bis 3, dadurch gekennzeichnet, daß R¹ -CH₂CH₂-, -(CH₂)₃-, -CH₂CH(CH₃)- oder -CH₂CH₂OCH₂CH₂- und X NH₄ oder ein Alkalimetall ist.

5. Verfahren nach einem oder mehreren der Ansprüche 1 bis 4, dadurch gekennzeichnet, daß die Veresterung bei einer Temperatur von 200 bis 240 °C durchgeführt wird.

6. Verfahren nach einem oder mehreren der Ansprüche 1 bis 5, dadurch gekennzeichnet, daß die Fettsäure und das Hydroxyalkansulfonat im Molverhältnis von 1 : 1 bis 2 : 1 eingesetzt werden.

7. Verfahren nach einem oder mehreren der Ansprüche 1 bis 5, dadurch gekennzeichnet, daß die Fettsäure und das Hydroxyalkansulfonat im Molverhältnis von etwa 1 : 1 eingesetzt werden.

8. Verfahren nach einem oder mehreren der Ansprüche 1 bis 7, dadurch gekennzeichnet, daß als Veresterungskatalysator Zinkoxid in einer Menge von 0,05 bis 2 Gew.-%, bezogen auf Fettsäure und Hydroxyalkansulfonat, eingesetzt wird.

9. Verfahren nach Anspruch 1, dadurch gekennzeichnet, daß man mindestens eine Fettsäure der Formel 1, worin R ein verzweigter Rest der nachstehenden Formel 1a ist 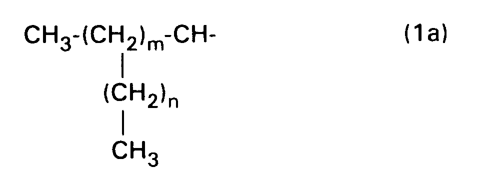 worin m eine ganze Zahl von 4 bis 18 und n 0 oder eine ganze Zahl von 1 bis 10 ist, mit mindestens einem Hydroxyalkansulfonat der Formel 2, worin R¹ -CH₂CH₂-, -(CH₂)₃-, -CH₂CH(CH₃)- oder -CH₂CH₂OCH₂CH₂- und X NH₄ oder ein Alkalimetall ist, im Molverhältnis von 1 : 1 bis 2 : 1 in Gegenwart von Zinkoxid in einer Menge von 0,05 bis 1 Gew.-%, bezogen auf Fettsäure und Hydroxyalkansulfonat, bei einer Temperatur von 200 bis 240 °C verestert.

10. Verfahren nach Anspruch 9, dadurch gekennzeichnet, daß man die Fettsäure und das Hydroxyalkansulfonat im Molverhältnis von etwa 1 : 1 einsetzt.
